# EUROPEAN PATENT APPLICATION

(11) **EP 4 641 543 A1**
(43) Date of publication of application: **29.10.2025**
(21) Application number: 23905818.3
(22) Date of filing: 15.12.2023
(51) Int. Cl.: G09B 9/00, A61C 9/00, G09B 5/02, G06F 9/451, G06T 19/00

(54) **SCANNING GUIDE METHOD, APPARATUS AND SYSTEM, MEDIUM, AND COMPUTER DEVICE**

(30) Priority: 22.12.2022 CN 202211659342
(71) Applicant: Shining 3D Tech Co., Ltd., Hangzhou, Zhejiang 311258 (CN)
(72) Inventor: YAN, Kexin, Hangzhou, Zhejiang 311258 (CN); ZHAO, Xiaobo, Hangzhou, Zhejiang 311258 (CN); ZHANG, Huiquan, Hangzhou, Zhejiang 311258 (CN); YING, Jiawei, Hangzhou, Zhejiang 311258 (CN); MA, Chao, Hangzhou, Zhejiang 311258 (CN)
(74) Representative: Metida
(86) International application number: PCT/CN2023/138999
(87) International publication number: WO 2024/131654

(57) **Abstract**

A scanning guidance method, an apparatus and a system, a medium and a computer device are provided. A target guidance path (R) is determined based on real-time model data (D) obtained by scanning a standard model (M) by a scanner (101), the target guidance path (R) is displayed in a three-dimensional digital model (m) corresponding to the standard model (M), thereby indicating a scanning method of the scanner (101) for the standard model (M). In this way, a user can intuitively view the scanning method for scanning the standard model (M) by observing the target guidance path (R), thereby improving an intuitiveness of a scanning guidance.

## Description

### TECHNICAL FIELD

The present disclosure relates to a field of data processing technology, and in particular to a scanning guidance method, an apparatus and a system, a medium and a computer device.

### BACKGROUND

At present, scanners are used more and more widely. Software of the scanners usually use some text, pictures and animation videos to guide users to operate the scanners. However, a guidance method through text, pictures and animation videos is not intuitive and effective enough.

### SUMMARY

In a first aspect, an embodiment of the present disclosure provides a scanning guidance method, which is applied to a processing unit, and the processing unit is connected to a scanner and a display unit in communication; the method includes: obtaining a three-dimensional digital model in a practice mode; obtaining real-time model data by scanning a standard model corresponding to the three-dimensional digital model using the scanner; determining a target guidance path based on the real-time model data, and the target guidance path being used to indicate a scanning method of the scanner for the standard model; controlling the display unit to display the three-dimensional digital model and display the target guidance path in the three-dimensional digital model.

In a second aspect, an embodiment of the present disclosure provides a scanning guidance apparatus, which is applied to a processing unit, and the processing unit is connected to a scanner and a display unit in communication; the apparatus comprises: a first acquisition module, used to obtain a three-dimensional digital model in a practice mode; a second acquisition module, used to obtain real-time model data by scanning a standard model corresponding to the three-dimensional digital model using the scanner; a control module, used to determine a target guidance path based on the real-time model data, and the target guidance path being used to indicate a scanning method of the scanner for the standard model; and control the display unit to display the three-dimensional digital model and display the target guidance path in the three-dimensional digital model.

In a third aspect, an embodiment of the present disclosure provides a scanning guidance system, comprising: a scanner for scanning a standard model in a practice mode to obtain real-time model data; a processing unit for determining a target guidance path based on the real-time model data, wherein the target guidance path is used to indicate a scanning method of the scanner for the standard model; and a display unit for displaying the target guidance path in a three-dimensional digital model corresponding to the standard model.

In a fourth aspect, an embodiment of the present disclosure provides a computer-readable storage medium having a computer program stored thereon, which when executed by a processor, implements the method described in any embodiment of the present disclosure.

In a fifth aspect, an embodiment of the present disclosure provides a computer device, comprising a storage device, a processor, and a computer program stored in the storage device and executable on the processor, wherein the processor implements the method described in any embodiment of the present disclosure when executing the program.

It is to be understood that both the foregoing general description and the following detailed description are exemplary and explanatory only and are not restrictive of the present disclosure.

### BRIEF DESCRIPTION OF THE DRAWINGS

The drawings herein are incorporated into the specification and constitute a part of the specification. These drawings illustrate embodiments consistent with the present disclosure and are used to illustrate the technical solutions of the present disclosure together with the specification.
FIG. 1 is a schematic diagram of an application scenario provided by an embodiment of the present disclosure.
FIG. 2 is a flow chart of a scanning guidance method provided by an embodiment of the present disclosure.
FIG. 3 is a schematic diagram of a method of entering a practice mode provided by an embodiment of the present disclosure.
FIG. 4 is a schematic diagram of a standard model provided by an embodiment of the present disclosure.
FIG. 5 is a schematic diagram of a guidance path of a standard model provided by an embodiment of the present disclosure.
FIG. 6 is a schematic diagram of an initial scanning path provided by an embodiment of the present disclosure.
FIG. 7 is a schematic diagram of a real-time scanning position provided by an embodiment of the present disclosure.
FIG. 8 is a schematic diagram of a rendering effect of real-time model data provided by an embodiment of the present disclosure.
FIG. 9 is a schematic diagram of a practice method for scanning a complete maxillomandibular provided by an embodiment of the present disclosure.
FIG. 10 is a flow chart of a practice process for scanning a maxillary provided by an embodiment of the present disclosure.
FIG. 11 is a flow chart of a practice process for scanning a complete maxillomandibular provided by an embodiment of the present disclosure.
FIG. 12 is a schematic diagram of a scanning guidance apparatus provided by an embodiment of the present disclosure.
FIG. 13 is a schematic diagram of a computer device provided by an embodiment of the present disclosure.

### DETAILED DESCRIPTION

Exemplary embodiments will be described in detail herein, examples of which are shown in the accompanying drawings. When the following description refers to the drawings, the same numbers in different drawings represent the same or similar elements unless otherwise indicated. The embodiments described in the following exemplary embodiments do not represent all embodiments consistent with the present disclosure. Instead, they are merely examples of devices and methods consistent with some aspects of the present disclosure as detailed in the appended claims.

Terms used in this disclosure are only for the purpose of describing specific embodiments, and are not intended to limit the disclosure. The singular forms of "a", "said" and "the" used in this disclosure and the appended claims are also intended to include plural forms, unless the context clearly indicates other meanings. It should also be understood that the term "and/or" used in this article refers to and includes any or all possible combinations of one or more associated listed items. In addition, the term "at least one" herein means any combination of at least two of any one or more of a plurality of.

It should be understood that although the terms first, second, third, etc. may be used in the present disclosure to describe various information, such information should not be limited to these terms. These terms are only used to distinguish the same type of information from each other. For example, without departing from the scope of the present disclosure, the first information may also be referred to as the second information, and similarly, the second information may also be referred to as the first information. Depending on the context, the word "if" as used herein may be interpreted as "at the time of" or "when" or "in response to determining".

In order to enable those skilled in the art to better understand the technical solutions in the embodiments of the present disclosure and to make the above-mentioned purposes, features and advantages of the embodiments of the present disclosure more obvious and understandable, the technical solutions in the embodiments of the present disclosure are further described in detail below in conjunction with the accompanying drawings.

With the continuous promotion and popularity of digital oral restoration technology, scanners have developed rapidly and become popular in recent years. More and more hospitals and clinics have begun to use scanners to take digital impressions of patients.

The scanner obtains a three-dimensional (3D) digital model of teeth and gums in an oral cavity by scanning and splicing local areas. When using the scanner to scan, there are high requirements for the method of use: the scanner needs to be placed at a certain position of the teeth in the mouth and stay for a while. After scanning 3D data of local teeth, the scanner is moved steadily to other areas of the teeth. During the movement, a scanning head needs to be kept close to a surface of the teeth (or gums) to gradually scan 3D data of other areas; if the scanning head moves too fast or leaves the surface of the teeth (or gums), a scanning may be interrupted. The user needs to move the scanning head back to a region that has been scanned and realign it before continuing to scan. If the scanning process is frequently interrupted, it will affect a speed and an efficiency of taking a mold; in addition, an data splicing effect (a success rate, an error, etc.) during a scanning process is closely related to a scanning technique (a posture and an orientation of the scanning head) and a path (a movement path of the scanning head). A good scanning technique and a path can achieve twice the result with half the effort, and a complete and effective high-quality 3D digital model of the oral cavity can be obtained quickly and smoothly in one scan.

However, most nurses in hospitals and clinics, especially those who have just started using scanners, are not familiar with and do not understand the above-mentioned usage skills of the scanners, nor do they have an intuitive understanding of good scanning methods, which makes it difficult for them to quickly get started and use the scanners proficiently.

Therefore, software of the scanner uses some text, pictures and animation videos to guide users to operate the scanner. However, guidance methods through text, pictures and videos are not intuitive and effective. They can only output information in a one-way way, allowing users to roughly understand the scanning skills, but cannot intuitively let users understand whether their scanning techniques are correct and efficient. It is also not conducive to users to improve and enhance their scanning skills, resulting in scanning data with poor quality, inaccurate bite, low scanning efficiency and other problems for a long time for novice users.

In a field of oral restoration, a quality of 3D data and an accuracy of occlusion are crucial for diagnosis and restoration of oral problems, and even directly affect a suitability of a restoration made by the technician. If the data quality is not high, the restoration made may not meet patient's requirements or even be completely unusable. Therefore, there is an urgent need for a more effective off-site, interactive software automation guidance for novice users to practice software functions.

Based on this, the present application provides a standard practice scanning method, which guides novice users to practice through a mechanism of real-time guidance, operation and feedback, and understands shortcomings of their scanning techniques based on real-time interactive feedback and evaluation after scanning, so as to facilitate novice users to make improvements and corrections, allowing novice users to become familiar with and master relevant scanning techniques more quickly, helping novice users to develop good scanning habits faster and better, making scanning more efficient and the scanned data quality higher.

Of course, the practice scanning method is not limited to be applied in the scanners described above, but can also be applied to 3D scanners in other application scenarios, such as handheld industrial 3D scanners for scanning parts.

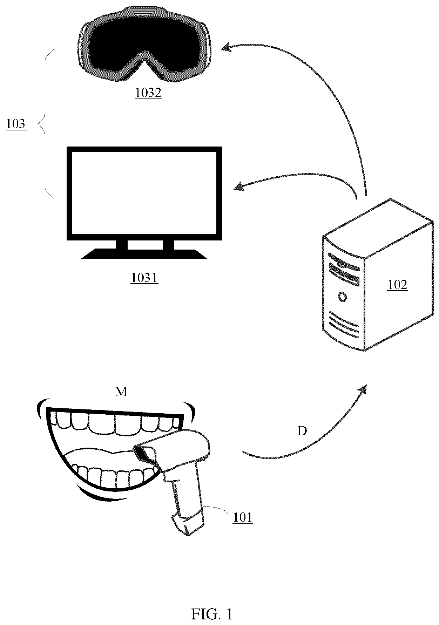

Solutions provided by the present disclosure are illustrated below. As shown in FIG. 1, it is a schematic diagram of an application scenario provided by the embodiment of the present disclosure, and the application scenario includes a scanner 101, a processing unit 102, and a display unit 103. Among them, the scanner 101 can be connected to the processing unit 102 and the display unit 103 in communication, and a connection method includes, but is not limited to, various wired or wireless connection methods such as a universal serial bus (USB) connection and a BLUETOOTH connection. The processing unit 102 may include, but is not limited to, a central processing unit (CPU), etc. The display unit 103 may include, but is not limited to, at least one of a display screen 1031 and augmented reality (AR) glasses 1032. The scanner 101 of the embodiment of the present disclosure may be an intraoral scanner (also called as "digital impression devices" or "inlet three-dimensional scanner"), or may be other types of scanners; a standard model M of the embodiment of the present disclosure may be a standard oral model, or may be other types of standard models. For ease of understanding, the following is an example in which the scanner 101 is an intraoral scanner and the standard model M is a standard oral model. When a user (e.g., a doctor, a nurse, etc.) is practicing scanning, the user can operate the scanner 101 to scan the standard model M through the scanner 101 to obtain real-time model data (e.g., real-time point cloud data of a surface of the standard model M) D of the standard model M. For example, when scanning, the scanner 101 can obtain image data of the standard model M at a certain frequency, and send the image data to the processing unit 102, and the processing unit 102 obtains local three-dimensional data of each photographing position on the surface of the standard model M through reconstruction, that is, single-frame point cloud data (also called as "single-frame data" or "scanned single frame"). After receiving the real-time model data D, the processing unit 102 can send control information to the display unit 103 to display scanning guidance information through the display unit 103. In this way, the user can intuitively observe the scanning guidance information on the display unit 103, thereby determining the scanning method for the standard model M, which improves the intuitiveness of the scanning guidance. Further, the application scenario can also include an interactive unit (not shown), such as a mouse, a keyboard, a voice interaction component, a touch screen, etc., for performing various human-computer interaction operations involved in a scanning guidance process.

The scanning guidance method of the embodiment of the present disclosure is described with reference to the application scenario shown in FIG. 1 . It is understood that the application scenario shown in FIG. 1 is only an exemplary description and is not intended to limit the present disclosure. In addition to the application scenario, the scanning guidance method of the embodiment of the present disclosure can also be used in other application scenarios.

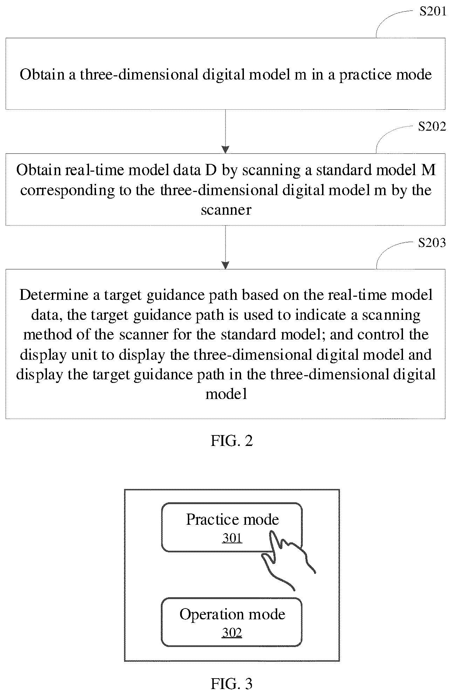

As shown in FIG. 2, the scanning guidance method of the embodiment of the present disclosure includes the following steps S201-S203:

Step S201: a three-dimensional digital model m is obtained in a practice mode;

Step S202: real-time model data D is obtained by scanning a standard model M corresponding to the three-dimensional digital model m using the scanner 101;

Step S203: a target guidance path R is determined based on the real-time model data D, the target guidance path R is used to indicate a scanning method of the scanner 101 for the standard model M; controlling the display unit 103 to display the target guidance path R in the three-dimensional digital model m corresponding to the standard model M.

The disclosed embodiment can determine the target guidance path based on the real-time model data, and display the target guidance path in the three-dimensional digital model. For example, before an initial scan, the displayed target guidance path can be an initial scanning path. During a real-time scanning process, it can be determined whether a model area that is currently scanned in the standard model has been scanned based on the real-time model data. If so, the target guidance path is updated to guide the user to scan a next model area in the standard model; if the currently scanned model area has not been scanned, the target guidance path is not updated to guide the user to continue scanning the currently scanned model area. The above process is executed in a loop until an entire standard model is scanned or the user manually exits the scanning process. Since the scanning process can provide a visual guidance based on the target guidance path, the intuitiveness of the scanning guidance is effectively improved.

The method of this embodiment can be executed in the practice mode. Scanning software can be deployed in the processing unit 102 to execute the method of this embodiment. A function entry 301 corresponding to the practice mode can be specifically provided in the scanning software, as shown in FIG. 3. After clicking the function entry 301 corresponding to the practice mode, the practice mode can be entered to practice scanning the standard model M, and the scanning practice process can be guided by the method of this embodiment. In addition to the practice mode, the scanning software can also provide function entries corresponding to other modes, for example, a function entry 302 corresponding to an operation mode. After clicking the function entry 302 corresponding to the operation mode, the operation mode can be entered to scan a real scan object. Among them, the real scan object can be an object of the same category as the standard model M. For example, when the standard model M is a standard oral model, the real scan object can be an oral cavity. The real scan data obtained by scanning can be point cloud data generated based on images of the real scan object, and the point cloud data can be displayed on the display unit.

In some embodiments, a functional interface of the operation mode and a function interface of the practice mode are at least partially different. For example, the functional interface of the operation mode includes the target guidance path R and the standard model M, while the functional interface of the operation mode does not include the target guidance path R and the standard model M. For another example, the functional interface of the operation mode may include a function entry such as AI deleting miscellaneous data, while the functional interface of the practice mode may include or may not include the function entry such as AI deleting miscellaneous data. In addition, according to actual needs, the functional interface of the practice mode and the functional interface of the operation mode may also include other identical or different function entrances, which are not listed here one by one.

In addition to the above two functional entries, the scanning software of the embodiment of the present disclosure may also provide other functional entries, which are not listed here one by one.

In step S201, the standard model M may be a standard (resin or plaster) maxillary-mandibular model, which is used as an object for practicing scanning. For example, a plurality of maxillary-mandibular models with a same shape, a same size and a same precision may be produced in a same manner as standard models M, and each scanner 101 may be equipped with one standard model M. In addition, the scanning software may have a built-in three-dimensional digital model m corresponding to the standard model M. The three-dimensional digital model m may be generated by a model making and design software, and then may be printed out by a 3D printing technology, thereby obtaining the standard model M; or the standard model M may be generated first, and then the standard model M may be scanned to obtain the corresponding three-dimensional digital model m, and then the three-dimensional digital model m may be printed and copied by the 3D printing technology to obtain more standard models M.

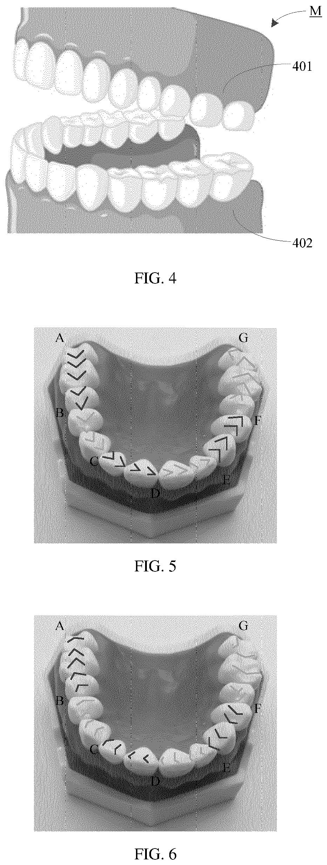

FIG. 4 shows a schematic diagram of a standard model M, which may include a maxillary model 401 and a mandibular model 402, where the maxillary model 401 and the mandibular model 402 may both include a tooth model and a gum model, and the tooth model includes a plurality of teeth. The figure shows a schematic diagram of the maxillary model 401 and the mandibular model 402 when they are not occluded. It can be understood that the maxillary model 401 and the mandibular model 402 may also be occluded. When the maxillary model 401 and the mandibular model 402 are not occluded, the standard model M is said to be in a non-occluded state; when the maxillary model 401 and the mandibular model 402 are occluded, the standard model M is said to be in an occluded state.

Furthermore, there may be a plurality of standard models M, and different standard models M may have different features, including a size of the standard model M, a number of teeth in the standard model M, a material, a degree of alignment, etc. Each scanner 101 may be equipped with the plurality of standard models M, so as to facilitate users to practice and adapt to various scanning scenarios.

As described above, the scanning software may have a built-in three-dimensional digital model m corresponding to the standard model M. After the scanning software is deployed to the processing unit 102, the processing unit 102 may invoke the display unit 103 to display the three-dimensional digital model m. After the scanner 101 starts scanning the standard model M, the processing unit 102 may also invoke the display unit 103 to display the model data actually scanned. The three-dimensional digital model m and the model data actually scanned may be displayed in different colors and/or transparencies to distinguish the three-dimensional digital model m from the model data actually scanned. For example, the three-dimensional digital model m may be displayed in a virtualized (a specific color such as gray and semi-transparent) manner, while the model data actually scanned may be displayed in a real color and opaque manner. For example, the model data corresponding to the teeth in the model data actually scanned may be displayed in a color corresponding to the teeth, and the model data corresponding to the gums may be displayed in a color corresponding to the gums. Different types of display units 103 may adopt different display methods. For example, when the display unit 103 is the display screen 1031 shown in FIG. 1, the image data of the three-dimensional digital model m and the scanned model data under a specific rendering camera perspective may be rendered; when the display unit 103 is the AR glasses shown in FIG. 1, the corresponding three-dimensional model may be rendered in an AR manner.

After obtaining the standard model M and its corresponding three-dimensional digital model m, a set of standard scanning paths can also be formulated, which is called the guidance path of the standard model M. The guidance path of the standard model M is used to describe a pose and a scanning path of the scanning head of the scanner 101 relative to the standard model M. The guidance path of the standard model M can be obtained from the scanning data recorded during the scanning process by a skilled scanner who performs a complete scanning operation on the standard model M according to a standard method. Because the scanning data records a position and an orientation of each scanned single frame relative to a current scanned model, the current scanned model and the standard model M are spliced and aligned using 3D software or tools, that is, the actual scanning path information (including information such as the position and orientation of the scanning head) can be converted to a coordinate system of the standard model M to obtain the guidance path of the standard model M. Furthermore, the recorded scanning data can also be adjusted and optimized appropriately using 3D software or tools, such as removing or adjusting the position and orientation of some bad path points (for example, jitter points caused by operating errors during the scanning process).

After obtaining the guidance path of the standard model M, the guidance path of the standard model M can also be divided into multiple sub-paths. A purpose of dividing the guidance path is to facilitate tracking a user's scanning and updating the guidance path. Generally, each sub-path can be equivalent to a coverage area of a single scan frame. As shown in Figure 5, the guidance path of the standard model M is divided into six sub-paths: AB, BC, CD, DE, EF, and FG. Each sub-path can include some scanning path information on the guidance path of the standard model M, and can also include a bounding box of a model area covered by the sub-path in the standard model M. For easy distinction, adjacent sub-paths are marked with different colors. In addition, in order to maintain a continuity and a transition of updating guidance, two adjacent sub-paths can partially overlap.

The guidance path of the standard model M may include an initial guidance path (for example, one of the multiple sub-paths may be determined as the initial guidance path), that is, a starting scanning path for scanning the standard model M. By setting the initial guidance path, the user may be restricted to scan the standard model M from a preset position. In order to meet different habits of different users, multiple initial guidance paths may be set. For example, the sub-path AB in FIG. 5 may be set as the initial guidance path, and the sub-path GF in FIG. 6 may be set as the initial guidance path. In actual applications, the initial guidance path may be set by the user through the interaction unit. Furthermore, a default initial guidance path may be set, and the user may switch the default initial guidance path through the interaction unit.

During the scanning guidance process, the standard model M may be scanned using the scanner 101, and model data obtained by scanning real-time may be obtained, namely, real-time model data D. The real-time model data D may include point cloud data of multiple points on the surface of the standard model M. The multiple points may include points on the teeth and/or gums of the standard model M.

In some embodiments, it is also possible to determine whether a current oral cavity model scanned using the scanner 101 is a standard model. Specifically, initial model data (for example, some real-time model data D obtained after the scanning starts) obtained by scanning the current oral cavity model using the scanner 101 can be obtained, and the initial model data can be spliced with the three-dimensional digital model m corresponding to the standard model M. If the splicing is successful, it is determined that the current oral cavity model is the standard model M; otherwise, it is determined that the current oral cavity model is not the standard model M. If the user scans a model other than the standard model, the processing unit 102 can output a prompt message to prompt the user to scan the standard model M according to a guided mode.

In step S202, the target guidance path R may be determined based on the real-time model data D. The target guidance path R is used to indicate a scanning method of the scanner 101 for the standard model M, where the scanning method includes, but is not limited to, a scanning position and orientation. By determining the target guidance path R, it is possible to indicate which position of the standard model M the scanning head of the scanner 101 is aligned with and how the scanning head moves during the scanning process. The target guidance path R displayed in the initial state may be the initial guidance path of the standard model M. As the scanning position is updated, the target guidance path R may be updated on the basis of the initial guidance path until the scanner 101 completes scanning the standard model M or the user manually stops the scanning practice process. The initial state may refer to a state where the scanning has not yet started, that is, the scanner 101 has not yet scanned the model data of the standard model M. That is, before the scanning starts, the standard model M and the target guidance path R in the initial state (i.e., the initial guidance path) may be displayed on the display unit 103, and after the scanning starts, the target guidance path R may be updated according to a real-time scanning position. The target guidance path R may be one of the multiple sub-paths included in the guidance path of the standard model.

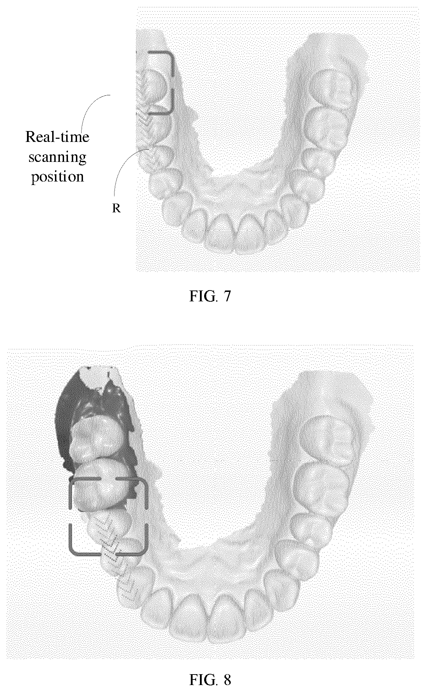

After the real-time model data D is initially acquired, the initial scanning area of the scanner 101 for the standard model M can be determined based on the real-time model data D initially acquired, and then it can be determined whether the initial scanning area matches a scanning area indicated by the initial guidance path. Specifically, the standard model M and its guidance path can be converted to a current scanning coordinate system, and it can be determined whether the initial scanning area matches the scanning area indicated by the initial guidance path in the current scanning coordinate system. If an overlap between the initial scanning area and the scanning area indicated by the initial guidance path reaches a preset overlap threshold, it can be determined that the two are matched, otherwise it is determined that the two are not matched. If the two are not matched, the processing unit 102 can output a prompt message to prompt the user to scan the standard model M according to a guided mode. If the two are matched, an operation of determining the target guidance path R based on the real-time model data D can be performed, and the real-time model data D can also be rendered (for example, the real-time model data is rendered on the three-dimensional digital model m), and a rendering result is displayed on the display unit 103. A rendering effect is shown in FIG. 8, wherein the standard model M and the real-time model data D can be rendered in different colors, so that the model area that has been scanned on the standard model M can be intuitively observed.

In some embodiments, a scanning progress of the current scanning area on the standard model M can be determined based on the real-time model data D; and the target guidance path R can be determined based on the scanning progress. For example, a first area S1 of a model bounding box corresponding to the current guidance path can be determined; a second area S2 of a region that has been scanned in the model bounding box can be determined based on the real-time model data D; and the scanning progress of the current scanning area can be determined based on a ratio of the second area S2 to the first area S1.

If the scanning progress does not reach a preset progress threshold, the current guidance path may be determined as the target guidance path R. If the scanning progress reaches the preset progress threshold, the current guidance path may be updated to obtain an updated guidance path, and the updated guidance path may be determined as the target guidance path R. Furthermore, if the scanning progress reaches the preset progress threshold, prompt information (e.g., text or audio prompt information) may be output to prompt the user to continue scanning according to the target guidance path R.

In the above embodiment, the current guidance path is a guidance path for guiding the user to move the scanner 101 to a current scanning area. The target guidance path R is a guidance path for guiding the user to move the scanner 101 to a next scanning area. Since the current scanning area and the next scanning area may be the same or have a high degree of overlap, the current guidance path and the target guidance path R may also be the same. In the case where the scanning path of the standard model M is divided into multiple sub-paths, the current guidance path may be one of the multiple sub-paths. When the scanning progress does not reach the preset progress threshold, the target guidance path R is the current guidance path. When the scanning progress reaches the preset progress threshold, the target guidance path R is a next path of the current guidance path. At this time, the current guidance path can be updated by hiding the current guidance path and displaying the next sub-path of the current guidance path, the updated guidance path is the next sub-path of the current guidance path. For example, in the embodiment shown in Figure 5, if the current guidance path is a sub-path AB and the target guidance path R is sub-path BC, before the current guidance path is updated, only the sub-path AB can be displayed without displaying other sub-paths; after the current guidance path is updated, the sub-path AB can be hidden and a sub-path BC can be displayed.

In step S203, the display unit 103 may be invoked to display the three-dimensional digital model m, and the target guidance path R may be displayed on the three-dimensional digital model m. A method for displaying the target guidance path R is shown in FIG. 5 and FIG. 6, where an arrow direction indicates the orientation of the scanning head of the scanner 101, and a position of the arrow indicates the scanning position of the scanning head of the scanner 101. In other embodiments, the target guidance path R may also be represented by multiple path points, each of which indicates a scanning position of the scanning head of the scanner 101, and the multiple path points together constitute the target guidance path R.

Furthermore, the display unit 103 can also be controlled to display a real-time scanning position of the scanner 101 on the standard model M in the three-dimensional digital model m. Referring to FIG. 7, the real-time scanning position can be a coverage area of single frame data scanned real-time, as shown by a rectangular box in the figure. As shown in FIG. 7, the real-time scanning position and the target guidance path R are simultaneously displayed in the three-dimensional digital model m.

In some embodiments, the real-time model data D can be spliced with historical model data obtained by scanning the standard model M using the scanner 101; if the splicing is successful, the display unit 103 is controlled to display the real-time scanning position in the three-dimensional digital model m. Specifically, if the splicing is successful, the pose of the single frame data scanned real-time can be determined, thereby determining the pose of the scanning head (i.e., the path points current scanned), and determining the real-time scanning position based on the pose of the scanning head. If the splicing fails, it is possible that the model area currently scanned by the user does not overlap enough with the scanned model area, and the position of the current scanned single frame cannot be located, and thus the position of the scanning head cannot be determined, and therefore, the real-time scanning position is not displayed. Furthermore, in the case of a splicing failure, a first prompt message can also be output to prompt the scanner 101 to return to the region that has been scanned on the standard model M.

In other embodiments, when it is not determined whether the real-time model data D and the historical model data are successfully spliced, one display mode can be used to display the real-time scanning position. When the real-time model data D and the historical model data are successfully spliced, another display mode can be used to display the real-time scanning position. For example, after obtaining the real-time scanning position, the real-time scanning position may be displayed in a flashing display mode first, and if the real-time model data D and the historical model data are successfully spliced, the display mode of the real-time scanning position is switched from the flashing display mode to a non-flashing display mode. For another example, after obtaining the real-time scanning position, the real-time scanning position may be displayed in a semi-transparent display mode first, and if the real-time model data D and the historical model data are successfully spliced, the display mode of the real-time scanning position is switched from the semi-transparent display mode to an opaque display mode. In addition to the display modes listed above, other display modes may also be used, which are not listed here one by one.

In some embodiments, the real-time model data D can be matched with the current guidance path; if the match is successful, the display unit 103 can be controlled to display the real-time scanning position of the scanner 101 on the standard model M in the three-dimensional digital model m. If the match fails, a second prompt message is output to prompt the scanner 101 to scan according to the guidance path. Among them, conditions for successful matching can be set according to actual conditions. For example, if the real-time model data D is within a guide area corresponding to the current guidance path (for example, the model bounding box corresponding to the current guidance path), it is determined that the match is successful, otherwise it is determined that the match fails.

The above-mentioned various prompt information (for example, the first prompt information and the second prompt information) may include visual prompt information such as text prompt information and icon prompt information, and may be output through the display unit 103, or may include audio prompt information, and may be output through an audio output unit. In the case where both the visual prompt information and the audio prompt information are included, the visual prompt information and the audio prompt information may be output synchronously. The user may set an on state and an off state of the visual prompt information and/or the audio prompt information through the interactive unit, thereby selecting different prompt modes.

The method of the above embodiment can be an oral scanning guidance method, which is used to guide and practice the scanning process of the intraoral scanner. Among them, the practice mode includes at least one of a maxillary scanning practice mode, a mandibular scanning practice mode and an occlusal scanning practice mode. **In** the maxillary scanning practice mode, the three-dimensional digital model m is a three-dimensional digital maxillary model, the standard model M is a standard maxillary model in a non-occlusal state, and the real-time model data D is real-time maxillary model data. **In** the mandibular scanning practice mode, the three-dimensional digital model m is a three-dimensional digital mandibular model, the standard model M is a standard mandibular model in a non-occlusal state, and the real-time model data D is real-time mandibular model data. **In** the occlusal scanning practice mode, the three-dimensional digital model m is a three-dimensional digital occlusal model of the three-dimensional digital maxillary model and the three-dimensional digital mandibular model in the occlusal state, the standard model M is a standard occlusal model of the standard maxillary model and the standard mandibular model in the occlusal state, and the real-time model data D is the real-time occlusal model data.

In the occlusal scanning practice mode, the maxillary model data obtained by scanning the standard maxillary model in the non-occlusal state using the scanner 101 in the maxillary scanning practice mode can be obtained, and the mandibular model data obtained by scanning the standard mandibular model in the non-occlusal state using the scanner 101 in the mandibular scanning practice mode can be obtained. Then, the real-time occlusal model data is spliced with the maxillary model data and/or the mandibular model data. The maxillary model data is data accumulated and spliced by multiple frames of real-time maxillary model data in the maxillary scanning practice mode, and the mandibular model data is data accumulated and spliced by multiple frames of real-time mandibular model data in the mandibular scanning practice mode. Taking the maxillary model data as an example, in the maxillary scanning practice mode, assuming that the real-time maxillary model data scanned at different times are recorded as {d1, d2,..., dk}, the maxillary model data is the data accumulated and spliced by d1, d2,..., dk. In some embodiments, d1, d2,..., dk may include data obtained after scanning the entire standard maxillary model. The method for obtaining the mandibular model data is similar to that for obtaining the maxillary model data, and will not be described in detail here.

Since the maxillary model data and the mandibular model data are obtained by scanning when the standard model M is in a non-occlusal state, there is no occlusal relationship between the maxillary model data and the mandibular model data. In the occlusal scanning practice mode, model data of the standard occlusal model at an occlusal position cannot be scanned, that is, the scanned model data is incomplete. Therefore, the embodiment of the present disclosure splices the real-time occlusal model data with the maxillary model data and/or the mandibular model data. On the one hand, it is possible to obtain the occlusal relationship between the maxillary model data and the mandibular model data; on the other hand, it is possible to scan relatively complete model data.

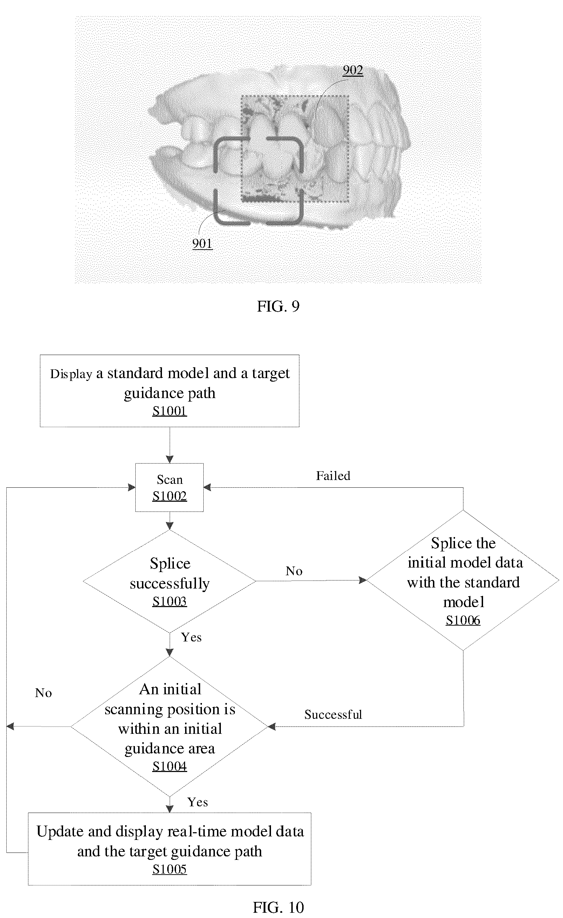

In some embodiments, the three-dimensional digital occlusal model and the real-time occlusal model data can also be spliced. If the splicing is successful, the real-time occlusal model data is spliced with the maxillary model data and/or the mandibular model data. If the splicing fails, the maxillary model data and/or the mandibular model data can be rescanned. As shown in Figure 9, if at least any one of the following conditions is met, it is determined that the three-dimensional digital occlusal model and the real-time occlusal model data are successfully spliced. A first condition: a portion of the three-dimensional digital maxillary model in the occluded state that is located within the preset scanning guide area 902 is successfully spliced with the real-time occlusal model data 901. A second condition: a portion of the three-dimensional digital mandibular model in the occluded state that is located within the preset scanning guide area 902 is successfully spliced with the real-time occlusal model data 901.

When any of the above conditions is met, the three-dimensional digital occlusal model can be displayed on the display unit 103, and when the splicing is successful, the maxillary model data and/or the mandibular model data can be displayed on the three-dimensional digital occlusal model. If both of the above conditions are not met, the maxillary model data and the mandibular model data can be discarded and not displayed. At the same time, a prompt message can also be output to prompt the user to scan the scanning guide area 902 according to the guidance.

The above-mentioned scanning guide area 902 can be preset, and the scanning guide area 902 can be a guiding area when the standard occlusal model is scanned for a first time. Accordingly, the maxillary model data and the mandibular model data can be the model data obtained by scanning the standard occlusal model for the first time. If the maxillary model data and the mandibular model data obtained by scanning the standard occlusal model for the first time meet any of the above conditions, when the standard occlusal model is scanned subsequently, the scanning area can no longer be guided and restricted (in accordance with an actual scanning situation, and the scanning area is relatively reasonable) for the user, and the user can scan relatively freely near the scanning guide area 902. In the process of scanning the standard occlusal model subsequently, the user can splice the real-time occlusal model data with the maxillary model data and/or the mandibular model data.

If the real-time occlusal model data is successfully spliced with the maxillary model data and/or the mandibular model data, the maxillary model data and/or the mandibular model data may be rendered on the three-dimensional digital occlusal model. Specifically, the three-dimensional digital occlusal model can be displayed in a virtualized manner. If the real-time occlusal model data is successfully spliced with the maxillary model data, and the real-time occlusal model data is successfully spliced with the mandibular model data, the maxillary model data and the mandibular model data can be rendered and displayed, and the corresponding three-dimensional digital maxillary model and the three-dimensional digital mandibular model can be hidden.

In some embodiments, even if the user scans according to the guidance path, due to an existence of errors, the model data obtained by the user's scan may have a certain deviation from the three-dimensional digital model corresponding to the standard model. Therefore, during the scanning process, if an amount of historical model data obtained using the scanner 101 scanning the standard model M reaches a preset data amount threshold, the historical model data can be aligned with the three-dimensional digital model to ensure correctness of scanning guidance instructions, judgments and feedback as much as possible. Specifically, iterative closest point (ICP) registration can be performed on multiple points in the historical model data and multiple points in the standard model, and the historical model data and the three-dimensional digital model can be aligned based on a registration result.

Through the scanning guidance method of the embodiment of the present disclosure, a separate scanning practice can be performed on a standard maxillary model or a standard mandibular model, or a scanning practice can be performed on an occlusal model. The overall flow of the two scanning practice processes is described below with reference to the accompanying drawings.

FIG. 10 is a scanning flow chart in the maxillary scanning practice mode. The process may include the following steps:

Step S1001, a standard oral cavity model M and a target guidance path R are displayed on the display unit 103, where the target guidance path R in an initial state is a preset initial guidance path.

Step S1002, the standard oral cavity model M is scanned through the intraoral scanner 101 to obtain real-time model data D. The real-time model data D obtained by an initial scanning may be referred to as initial model data.

Step S1003, whether the initial model data has been successfully spliced with the standard oral cavity model M is determined. If yes, execute step S1004; otherwise, execute step S1006.

Step S1004, whether an initial scanning position is within an initial guidance area (i.e., a scanning area corresponding to the initial guidance path) is determined. If yes, execute step S1005; otherwise, return to step S1002.

Step S1005, when a scanning progress reaches a progress threshold, real-time model data and the target guidance path R are updated and displayed, and the process returns to step S1002 until a maxillary scanning practice process of the standard model M is completed, or a user manually stops the maxillary scanning practice process.

Step S1006, the initial model data is spliced with the standard model M. If the splicing is successful, execute step S1004; otherwise, return to step S1002.

The scanning process in the mandibular scanning practice mode is similar to that in the maxillary scanning practice mode and will not be described in detail here.

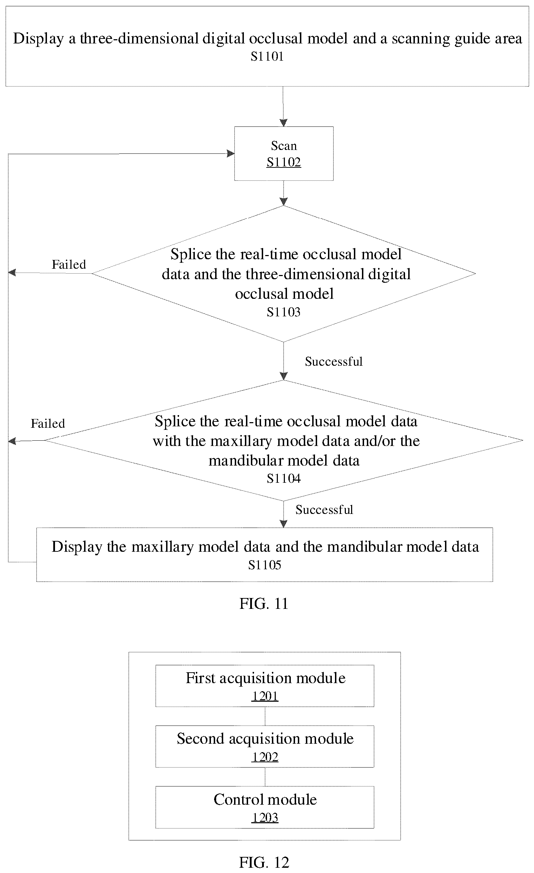

As shown in FIG. 11, it is a scanning flow chart in the complete maxillomandibular scanning practice mode. The process may include the following steps:

Step S1101, the three-dimensional digital occlusal model and the scanning guide area 902 are displayed on the display unit 103.

Step S1102, the standard occlusal model is scanned using the scanner 101 to obtain real-time occlusal model data.

Step S1103, whether the real-time occlusal model data and the three-dimensional digital occlusal model have been successfully spliced is determined. If yes, execute step S1104; otherwise, return to step S1102.

Step S1104, whether the real-time occlusal model data is successfully spliced with the maxillary model data and/or the mandibular model data is determined. If yes, execute step S1105; otherwise, return to step S1102.

Step S1105, the maxillary model data and the mandibular model data are displayed on the three-dimensional digital occlusal model.

When the maxillary model data and the mandibular model data are successfully spliced, the scanning practice process can be automatically completed. If the maxillary model data and the mandibular model data cannot be successfully spliced, the user can also manually complete the practice (for example, send a practice end instruction through the interactive unit) to enter subsequent practice results.

In some embodiments, the processing unit 102 may obtain scanning statistics in the practice mode, the scanning statistics are related to a scanning quality, and the scanning quality in the practice mode is scored based on the scanning statistics. The statistical data includes, but is not limited to, at least one of the following: a scanning time of the standard model, a matching degree between an actual scanning path of the standard model M and the guidance path of the standard model, a coverage completeness of the scanned model data on the standard model M, a matching degree between the scanned model data and the standard model M, and a matching degree between model data scanned for each part of the standard model M.

The actual scanning path of the standard model M may include an actual scanning path of the entire standard model M, and accordingly, the guidance path of the standard model may include the guidance path of the entire standard model M. Alternatively, the actual scanning path of the standard model M may include the actual scanning path of each model area on the standard model, and accordingly, the guidance path of the standard model may include the guidance path of each model area on the standard model. The coverage completeness of the model data obtained by scanning the standard model M may include the coverage completeness of the model data obtained by scanning the entire standard model M for the entire standard model M, and may also include the coverage completeness of the model data obtained by scanning each model area on the standard model M for the corresponding model area. The matching degree between the model data obtained by scanning and the standard model M may include the matching degree between the model data obtained by scanning the entire standard model M and the entire standard model M, and may also include the matching degree between the model data obtained by scanning each model area on the standard model M and the corresponding model area. The model data obtained by scanning each part in the standard model M may include the model data obtained by scanning the standard maxillary model in a non-occlusal state and the model data obtained by scanning the standard mandibular model in a non-occlusal state.

Furthermore, the scanning statistics in different practice modes can be obtained respectively. For example, in the maxillary scanning practice mode, the scanning time obtained is the scanning time of the standard maxillary model, in the mandibular scanning practice mode, the scanning time obtained is the scanning time of the standard mandibular model, and in the occlusal scanning practice mode, the scanning time obtained is the scanning time of the standard occlusal model. For another example, the matching degree between the actual scanning path of the standard model M and the guidance path of the standard model, the coverage completeness of the scanned model data on the standard model M, and the matching degree between the scanned model data and the standard model M can all include the corresponding data in the maxillary scanning practice mode, the mandibular scanning practice mode and the occlusal scanning practice mode. By scoring the scanning quality, the user can understand the practice situation. For example, multiple dimensions of scoring can be set, and each dimension corresponds to a scanning statistical data. The score of the practice can be obtained by combining the scores of multiple dimensions (for example, adding them up).

Furthermore, the processing unit 102 can save the scanning statistics of all the historical scanning practice processes of the user locally or upload them to a cloud platform for management. The scanning statistics of the user may be updated when each practice is completed, so that the user can understand a comparison of this practice in all his/her historical practice processes, for example, changes in various indicators such as the scanning time of the standard maxillary model and the scanning time of the standard mandibular model can be compared. The scanning statistics of each scanning practice process of the user can also be sorted, for example, the scanning time of the standard maxillary model of each scanning practice process of the user can be sorted, so that the user can understand the ranking of this scanning practice in the previous scanning practice processes and the overall practice situation and trend.

Furthermore, the cloud platform can summarize and rank in different dimensions based on the scanning statistics of all authorized users that are uploaded. After each practice, the software may guide the user to upload the scanning statistics and obtain the user's score ranking from the cloud, which increases the fun of scanning technique practice.

Referring to FIG. 12, an embodiment of the present disclosure further provides a scanning guidance apparatus, which is applied to a processing unit, and the processing unit is connected to a scanner and a display unit for communication; the apparatus comprises:

A first acquisition module 1201, which is used to acquire a three-dimensional digital model in a practice mode;

A second acquisition module 1202, which is used to acquire real-time model data by scanning a standard model corresponding to the three-dimensional digital model using the scanner;

A control module 1203, which is used to determine a target guidance path based on the real-time model data, where the target guidance path is used to indicate a scanning method of the scanner for the standard model; and control the display unit to display the three-dimensional digital model and display the target guidance path in the three-dimensional digital model.

In some embodiments, the functions or modules included in the device provided by the embodiments of the present disclosure can be used to execute the method described in the above method embodiments. The specific implementation can refer to the description of the above method embodiments, and for the sake of brevity, it will not be repeated here.

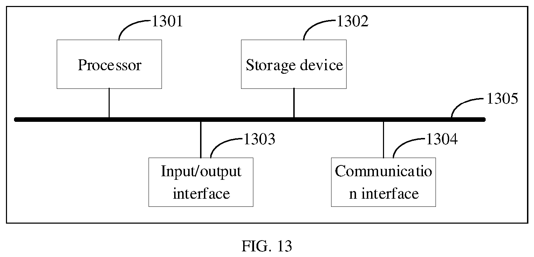

FIG. 13 shows a more specific schematic diagram of a hardware structure of a computing device provided by an embodiment of the present disclosure, and the device may include: a processor 1301, a storage device 1302, an input/output interface 1303, a communication interface 1304, and a bus 1305. The processor 1301, the storage device 1302, the input/output interface 1303, and the communication interface 1304 are connected to each other in communication within the device through the bus 1305.

The processor 1301 may be implemented by a general-purpose central processing unit (CPU), a microprocessor, an application-specific integrated circuit (ASIC), or one or more integrated circuits, and is used to execute relevant programs to implement the technical solutions provided by the embodiments of the present disclosure. The processor 1301 may also include a graphics card, which may include a Nvidia titan X graphics card or a 1080Ti graphics card.

The storage device 1302 may be implemented in the form of a read-only memory (ROM), a random access memory (RAM), a static storage device, a dynamic storage device, etc. The storage device 1302 may store an operating system and other application programs. When the technical solution provided by the embodiment of the present disclosure is implemented by software or firmware, the relevant program code is stored in the storage device 1302 and is invoked and executed by the processor 1301.

The input/output interface 1303 is used to connect an input/output module to realize information input and output. The input/output module can be configured in the device as a component (not shown in the figure), or it can be externally connected to the device to provide corresponding functions. The input device may include a keyboard, a mouse, a touch screen, a microphone, various sensors, etc., and the output device may include a display, a speaker, a vibrator, an indicator light, etc.

The communication interface 1304 is used to connect a communication module (not shown) to realize communication interaction between the device and other devices. The communication module can realize communication through wired means (such as USB, network cable, etc.) or wireless means (such as mobile network, WIFI, BLUETOOTH, etc.).

The bus 1305 includes a path for transmitting information between various components of the device (e.g., the processor 1301, the storage device 1302, the input/output interface 1303, and the communication interface 1304).

It should be noted that, although the device only shows the processor 1301, the storage device 1302, the input/output interface 1303, the communication interface 1304 and the bus 1305, in the specific implementation process, the device may also include other components necessary for normal operation. In addition, those skilled in the art can understand that the above device may also only include the components necessary for implementing the embodiments of the present disclosure, and does not necessarily include all the components shown in the figure.

The present disclosure also provides a scanning guidance system, the system includes:

A scanner 101, which is used to scan the standard model in the practice mode to obtain real-time model data;

A processing unit 102, which is configured to determine a target guidance path based on the real-time model data, where the target guidance path is used to indicate a scanning method of the scanner for the standard model;

A display unit 103, which is used to display the target guidance path in the three-dimensional digital model corresponding to the standard model.

A system architecture of the scanning guidance system can refer to the schematic diagram of the application scenario shown in Figure 1. The specific functions of each component under the system architecture are detailed in the above method embodiment, which will not be repeated here.

The present disclosure also provides a computer-readable storage medium on which a computer program is stored. When the program is executed by a processor, the method described in any of the above embodiments is implemented.

The computer-readable storage medium includes permanent and non-permanent, removable and non-removable medium that can be implemented by any method or technology to store information. Information can be computer-readable instructions, data structures, program modules or other data. Examples of computer storage medium include, but are not limited to, a phase change memory (PRAM), a static random access memory (SRAM), a dynamic random access memory (DRAM), other types of random access memory (RAM), read-only memory (ROM), electrically erasable programmable read-only memory (EEPROM), flash memory or other memory technology, compact disk read-only memory (CD-ROM), digital versatile disk (DVD) or other optical storage, magnetic cassettes, magnetic tape magnetic disk storage or other magnetic storage devices or any other non-transmission media that can be used to store information that can be accessed by a computing device. As defined in this article, computer-readable media does not include transitory computer - readable media such as modulated data signals and carrier waves.

It can be known from the above description of the implementation mode that the technicians in this field can clearly understand that the embodiments of this specification can be implemented by means of software plus a necessary general hardware platform. Based on such an understanding, the technical solution of the embodiments of this specification can be essentially or the part that contributes to the prior art can be embodied in the form of a software product, and the computer software product can be stored in a storage medium, such as ROM/RAM, a disk, an optical disk, etc., including a number of instructions for a computer device (which can be a personal computer, a server, or a network device, etc.) to execute the methods described in each embodiment or some parts of the embodiments of this specification.

The systems, devices, modules or units described in the above embodiments may be implemented by computer devices or entities, or by products with certain functions. A typical implementation device is a computer, which may be in the form of a personal computer, a laptop computer, a cellular phone, a camera phone, a smart phone, a personal digital assistant, a media player, a navigation device, an email transceiver, a game console, a tablet computer, a wearable device or a combination of any of these devices.

Each embodiment in this specification is described in a progressive manner, and the same and similar parts between the embodiments can be referred to each other, and each embodiment focuses on the differences from other embodiments. **In** particular, for the device embodiment, since it is basically similar to the method embodiment, the description is relatively simple, and the relevant parts can be referred to the partial description of the method embodiment. The device embodiment described above is merely schematic, wherein the modules described as separate components may or may not be physically separated, and the functions of each module can be implemented in the same one or more software and/or hardware when implementing the embodiment scheme of this specification. It is also possible to select some or all of the modules according to actual needs to achieve the purpose of the embodiment scheme. A person of ordinary skill in the art can understand and implement it without paying creative labor.

The above is only a specific implementation of the embodiments of this specification. It should be pointed out that for ordinary technicians in this technical field, several improvements and modifications can be made without departing from the principles of the embodiments of this specification. These improvements and modifications should also be regarded as the protection scope of the embodiments of this specification.

## Claims

1. A scanning guidance method applied to a processing unit, **characterized in that** the processing unit is connected to a scanner and a display unit for communication, the method comprises:
obtaining a three-dimensional digital model in a practice mode;
acquiring real-time model data by scanning a standard model corresponding to the three-dimensional digital model using the scanner;
determining a target guidance path based on the real-time model data, the target guidance path being used to indicate a scanning method of the scanner for the standard model; and controlling the display unit to display the three-dimensional digital model and display the target guidance path in the three-dimensional digital model.

2. The method according to claim 1, further comprising:
controlling the display unit to display a real-time scanning position of the scanner on the standard model in the three-dimensional digital model.

3. The method according to claim 2, wherein controlling the display unit to display the real-time scanning position of the scanner on the standard model in the three-dimensional digital model comprises:
splicing the real-time model data with historical model data obtained by scanning the standard model using the scanner;
in response that the splicing is successful, controlling the display unit to display the real-time scanning position in the three-dimensional digital model.

4. The method according to claim 3, further comprising:
in response that the splicing is failed, outputting a first prompt message, wherein the first prompt message is used to prompt the scanner to return to a region that has been scanned on the standard model.

5. The method according to claim 2, wherein controlling the display unit to display the real-time scanning position of the scanner on the standard model in the three-dimensional digital model comprises:
matching the real-time model data with a current guidance path;
in response that the matching is successful, controlling the display unit to display the real-time scanning position of the scanner on the standard model in the three-dimensional digital model.

6. The method according to claim 5, further comprising:
in response that the matching is failed, outputting a second prompt message, wherein the second prompt message is used to prompt the scanner to scan according to the target guidance path.

7. The method according to claim 1, wherein determining the target guidance path based on the real-time model data comprises:
determining a scanning progress of a current scanning area on the standard model based on the real-time model data;
determining the target guidance path based on the scanning progress.

8. The method according to claim 7, wherein determining the target guidance path based on the scanning progress comprises:
in response that the scanning progress does not reach a preset progress threshold, determining the current guidance path as the target guidance path.

9. The method according to claim 7, wherein determining the target guidance path based on the scanning progress comprises:
in response that the scanning progress reaches a preset progress threshold, updating the current guidance path to obtain an updated guidance path, and determining the updated guidance path as the target guidance path.

10. The method according to claim 9, wherein a scanning path of the standard model is divided into a plurality of sub-paths, and the current guidance path is one of the plurality of sub-paths; and updating the current guidance path to obtain the updated guidance path comprises:
hiding the current guidance path, and displaying a next sub-path of the current guidance path.

11. The method according to claim 7, wherein determining the scanning progress of the current scanning area on the standard model based on the real-time model data comprises:
determining a first area of a model bounding box corresponding to the current guidance path;
determining a second area of a region that has been scanned in the model bounding box based on the real-time model data;
determining the scanning progress of the current scanning area based on a ratio of the second area to the first area.

12. The method according to claim 1, further comprising:
acquiring initial model data by scanning a current oral model using the scanner;
splicing the initial model data with the three-dimensional digital model corresponding to the standard model;
in response that the splicing is successful, determining the current oral cavity model to be the standard model.

13. The method according to claim 1, further comprising:
rendering the real-time model data on the three-dimensional digital model.

14. The method according to claim 13, wherein rendering the real-time model data on the three-dimensional digital model comprises:
determining an initial scanning area of the scanner on the standard model;
in response that the initial scan area matches a scanning area indicated by the initial guidance path, rendering the real-time model data on the three-dimensional digital model.

15. The method according to claim 1, wherein the method is a scanning guidance method for an oral cavity, and the practice mode comprises at least one of a maxillary scanning practice mode, a mandibular scanning practice mode and an occlusal scanning practice mode;
in the maxillary scanning practice mode, the three-dimensional digital model is a three-dimensional digital maxillary model, the standard model is a standard maxillary model in a non-occlusal state, and the real-time model data is real-time maxillary model data;
in the mandibular scanning practice mode, the three-dimensional digital model is a three-dimensional digital mandibular model, the standard model is a standard mandibular model in a non-occlusal state, and the real-time model data is real-time mandibular model data;
in the occlusal scanning practice mode, the three-dimensional digital model is a three-dimensional digital occlusal model of a three-dimensional digital maxillary model and a three-dimensional digital mandibular model in an occlusal state, the standard model is a standard occlusal model of a standard maxillary model and a standard mandibular model in an occlusal state, and the real-time model data is real-time occlusal model data.

16. The method according to claim 15, further comprising:
in the occlusion scanning practice mode, obtaining maxillary model data by scanning the standard maxillary model in the non-occlusion state using the scanner in the maxillary scanning practice mode;
obtaining mandibular model data by scanning the standard mandibular model in the non-occlusal state using the scanner in the mandibular scanning practice mode;
splicing the real-time occlusion model data with the maxillary model data and/or mandibular model data.

17. The method according to claim 16, wherein splicing the real-time occlusion model data with the maxillary model data and/or mandibular model data comprises:
splicing the three-dimensional digital occlusal model with the real-time occlusal model data;
in response that the splicing is successful, splicing the real-time occlusal model data with the maxillary model data and/or the mandibular model data.

18. The method according to claim 17, further comprising: determining the three - dimensional digital occlusal model and the real-time occlusal model data are successfully spliced in response that at least any one of following conditions is met:
a portion of the three-dimensional digital maxillary model in the occlusal state that is located within a preset scanning guide area is successfully spliced with the real-time occlusal model data;
a portion of the three-dimensional digitized mandibular model in the occluded state that is located within the preset scanning guide area is successfully spliced with the real-time occlusal model data.

19. The method according to claim 1, further comprising:
in response that an amount of historical model data obtained by scanning the standard model using the scanner reaches a preset data amount threshold, aligning the historical model data with the three-dimensional digital model.

20. The method according to claim 1, further comprising:
obtaining real scan data by scanning a real scan object using the scanner in an operation mode;
controlling the display unit to display the real scan data;
wherein a functional interface of the operation mode and a functional interface of the practice mode are at least partially different.

21. The method according to claim 1, further comprising:
obtaining scanning statistics data in the practice mode; the scanning statistics data being related to a scanning quality;
scoring the scanning quality in the practice mode based on the scan statistics.

22. The method according to claim 21, wherein the scanning statistics comprises at least one of following:
a scanning time of the standard model, a matching degree between the actual scanning path of the standard model and the guidance path of the standard model, a complete coverage degree of the standard model by the scanned model data, a matching degree between the scanned model data and the standard model, and a matching degree between the model data obtained by scanning various parts of the standard model.

23. A scanning guidance apparatus applied to a processing unit, **characterized in that** the processing unit is connected to a scanner and a display unit for communication, the apparatus comprises:
a first acquisition module, being configured to acquire a three-dimensional digital model in a practice mode;
a second acquisition module, being configured to acquire real-time model data by scanning a standard model corresponding to the three-dimensional digital model using the scanner;
a control module, being configured to determine a target guidance path based on the real-time model data, wherein the target guidance path is used to indicate a scanning method of the scanner for the standard model; and control the display unit to display the three-dimensional digital model and display the target guidance path in the three-dimensional digital model.

24. A scanning guidance system, **characterized in that** the scanning guidance system comprises:
a scanner, being configured to scan a standard model in practice mode to obtain real-time model data;
a processing unit, being configured to determine a target guidance path based on the real-time model data, the target guidance path being used to indicate a scanning method of the scanner for the standard model;
a display unit, being configured to display the target guidance path in the three-dimensional digital model corresponding to the standard model.

25. A computer-readable storage medium having a computer program stored thereon, **characterized in that** when the program is executed by a processor, the method according to any one of claims 1 to 22 is implemented.

26. A computer device comprising a storage device, a processor and a computer program stored in the storage device and executable on the processor, **characterized in that** the processor implements the method according to any one of claims 1 to 22 when executing the program.
